# EUROPEAN PATENT SPECIFICATION

(11) **EP 1 163 519 B1**
(45) Date of publication and mention of the grant of the patent: **26.07.2006**
(21) Application number: 00911077.6
(22) Date of filing: 17.03.2000
(51) Int. Cl.: G01N 33/532, C12Q 1/37, C12N 15/10, C07K 16/00

(54) **PROTEIN ISOLATION AND ANALYSIS**
ISOLIERUNG UND ANALYSE VON PROTEINEN
ISOLEMENT ET ANALYSE DE PROTEINE

(30) Priority: 23.03.1999 GB 9906551; 29.03.1999 GB 9907057; 06.04.1999 GB 9907641; 28.06.1999 GB 9914874; 02.07.1999 GB 9915363; 06.07.1999 GB 9915677; 14.07.1999 GB 9916511; 31.08.1999 GB 9920503; 21.09.1999 GB 9922285
(43) Date of publication of application: 19.12.2001
(73) Proprietor: Biovation Limited, Aberdeen AB10 1DQ (GB)
(72) Inventor: CARR, Francis, J., Biovation Limited, Aberdeen AB22 8GU (GB)
(74) Representative: Benz, Jürgen
(86) International application number: PCT/GB2000/001015
(87) International publication number: WO 2000/057183

(56) References cited:
- WO-A-92/15679
- WO-A-95/16209
- J M KERR ET AL: "Encoded Combinatorial Peptide Libraries Containing Non-Natural Amino Acids" JOURNAL OF THE AMERICAN CHEMICAL SOCIETY,US,AMERICAN CHEMICAL SOCIETY, WASHINGTON, DC, vol. 115, no. 115, 24 March 1993 (1993-03-24), pages 2529-2531-2531, XP002128603 ISSN: 0002-7863
- NIKOLAIEV V ET AL: "PEPTIDE RESEARCH. PEPTIDE-ENCODING FOR STRUCTURE DETERMINATION OF NONSEQUENCEABLE POLYMERS WITHIN LIBRARIES SYNTHESIZED AND TESTED ON SOLID-PHASE SUPPORTS" PEPTIDE RESEARCH,US,NATICK, MA, vol. 6, no. 3, 1993, pages 161-170, XP000867524 ISSN: 1040-5704
- CAO P ET AL: "Analysis of Peptides, Proteins, Protein Digests, and Whole Human Blood by Capillary Electrophoresis/Electrospray Ionization-Mass Spectrometry Using an In-capillary Electrode Sheathless Interface" JOURNAL OF THE AMERICAN SOCIETY FOR MASS SPECTROMETRY,US,ELSEVIER SCIENCE INC, vol. 9, no. 10, 1 October 1998 (1998-10-01), pages 1081-1088, XP004140548 ISSN: 1044-0305
- VON LEOPRECHTING ACHIM; HOERTH PATRIC; HAEHNEL WOLFGANG; SCHILZ EMILE; MUEHLENHOFF ULRICH: "Identification of biotinylation sites on proteins by selective retrieval of 2-iminobiotinylated peptides from proteolytic peptide mixtures: Localization of the accessible lysine residues on the photosystem I subunits PsaD and PsaE" ANALYTICAL BIOCHEMISTRY, vol. 262, 10 September 1998 (1998-09-10), pages 110-121, XP002143247
- DUCRET AXEL; VAN OOSTVEEN INGE; ENG JIMMY K; YATES JOHN R III; AEBERSOLD RUEDI: "High throughout protein characterization by automated reverse-phase chromatography/electrospray tandem mass spectrometry" PROTEIN SCIENCE, vol. 7, March 1998 (1998-03), pages 706-719, XP000922504
- WANG BAIYANG; CHEN YI-BEN; AYALON ORAN; BENDER JEFFREY; GAREN ALAN: "Human single-chain Fv immunoconjugates targeted to a melanoma-associated chondroitin sulfate proteoglycan mediate specific lysis of human melanoma cells by natural killer cells and complement" PROCEEDINGS OF THE NATIONAL ACADEMY OF SCIENCES OF THE UNITED STATES OF AMERICA, vol. 96, 16 February 1999 (1999-02-16), pages 1627-1632, XP002143248
- GENG M ET AL: "Signature-peptide approach to detecting proteins in complex mixtures" JOURNAL OF CHROMATOGRAPHY,NL,ELSEVIER SCIENCE PUBLISHERS B.V. AMSTERDAM, vol. 870, no. 1-2, 18 February 2000 (2000-02-18), pages 295-313, XP004187331 ISSN: 0021-9673
- P L COURCHESNE ET AL: "Identification of Proteins by Matrix-Assisted Laser Desorption/Ionization Mass Spectroscopy Using Peptide and Fragment Ion Masses (from 2-D Proteome Analysis Protocols, Ed. A. J. Link)" METHODS IN MOLECULAR BIOLOGY,US,HUMANA PRESS INC., CLIFTON, NJ, vol. 112, no. 112, 1999, pages 487-511-511, XP002103063

## Description

The present invention relates to the isolation and analysis of proteins especially by mass analysis. The invention has particular application to the isolation of binding proteins such as antibodies.

For the isolation of proteins, the invention provides new methods for isolating specific proteins from a complex mixture of such proteins by virtue of binding to a specific target. In particular, the invention provides methods for isolating specific antibody domains from a gene library-derived mixture of such domains by virtue of binding to a specific target antigen. For the analysis of proteins the invention provides new methods for analysing complex mixtures of proteins especially to compare proteins between two or more different samples.

The invention relates, in particular, to specific libraries of individual proteins which comprise identifier sequence tracks by means of which the individual proteins can be identified. The invention relates, furthermore, to methods of indentification and screening of proteins which are members of said libraries. The invention relates also to methods for amplification and expression of the genes encoding an individual protein from said libraries.

For the isolation of proteins from complex mixtures by virtue of binding to a specific target and where the identity or amino acid sequence of the protein is unknown beforehand, it has usually been very difficult to isolate enough protein which binds to the target for direct characterisation of the protein. In order to select a protein of interest from a large library of natural, synthetic or semi-synthetic protein, "protein display" methods have been developed whereby recombinant proteins are produced physically linked to their genes such that recovery of the proteins allows subsequent rapid recovery of the genes. Such methods include "*in-vivo*" display methods such as display on bacteriophage ("phage display"), bacteria and yeast, and include *"in-vivo*" display methods such as display on ribosomes (''ribosome display"). The recovered genes can be sequenced in order to determine the identity of the recovered protein or can be used to regenerate the recovered protein. Document WO 9215679 discloses improved libraries of phage display which present various protein domains with potential to bind to a target material of interest wherein site-specific protease cleavable linkers may be incorporated. If a library of genes is subject to protein display methods whereby proteins are selected for a particular characteristics such as binding to an antigen (for antibody variable regions), then at each selection round, the recovered genes will be enriched for those encoding proteins exhibiting such particular characteristics. Disadvantage of current "*in-vivo*" display methods include a limit to the amount of functional protein displayed (phage display is usually limited to polypeptides of less than 40kDa), the usual need to fuse the recombinant protein to a host protein (which may interfere with the function or binding of the recombinant protein), and an inability to vary the number of proteins displayed per display particle; the latter is also a problem with "*in-vivo*" display methods such as ribosome display. In addition, methods for the selection of proteins with particular characteristics such as binding to an antigen are limited due to the small sizes of the display particles such that methods such as fluorescence activated cell sorting (FACS) cannot readily be used. Thus, there remains a need for new methods to improve the isolation of proteins from complex mixtures, in particular to improve the isolation of antibody variable regions (Fv's) from complex mixtures of Fv's. This, the present invention provides for improved methods for isolation of proteins from complex mixtures. In particular, the present invention combines the use of protein libraires generated from gene libraries with improvements in mass spectrometry and especially improvements in matrix-assisted laser desorption/ionisation time-of-flight (MALDI-ToF) spectromentry and the ability to directly sequence ToF-separated peptides by tandem mass spectrometry (MS-MS) and, more recently, the ability to combine ToF and MS/MS into one device (Q-ToF) and the ability to combine HPLC and electron sprat (ES) tandem mass spectrometry.

Thus, the present invention provides a method of protein identification, screening and/or sequencing comprising providing a library of individual proteins, one or more of which may bind to a target of interest, wherein each individual protein includes in its sequence a "barcode" sequence, which can be used to identify each individual protein in the library.

This aspect of the present invention provides for libraries of proteins, especially recombinant antibody domains such as Fv's, whereby individual protein members of the library include, within their amino acid sequence, a tract of sequence (a "barcode") which can subsequently be sequenced in order to identify which protein(s) has bound to the specific target (or, in the case of Fv's, "antigen"). This embodiment will apply especially where the Fv's are derived from human genes whereby the selected Fv may be suitable for human therapeutic or diagnostic use. In this particular application, an extensive gene library of Fv's is created from a pool of immunoglobulin cDNA's such as those derived from peripheral blood B cells in humans or such as pools created synthetically using human variable regions with semi-randomised ("combinatorial") CDRs (complimentarity-determining regions) at one or more positions. If this gene library is created in such manner that a random (or semi-random) gene sequence is included within the Fv coding region or terminal to this region, then such a random/semi-random gene sequence will generate a random/semi-random peptide sequence associated with individual Fv's. Such a random/semi-random gene sequence is created using standard methods such as oligonucleotide priming/DNA polymerase: extension or PCR whereby a random/semi-random synthetic oligonucleotide sequence is used as one of a pair of primers used to amplify immunoglobulin gene fragments during the creation of the Fv gene library. If members of the Fv library comprise two chains (i.e. heavy and light chain-derived chains (VH and VL)) as opposed to a single-chain (VH and VL joined by a peptide linker), then individual barcodes can be associated with each of the chains (or can be associated with one of the chains only). Upon creation of the library, the resultant Fv's each include one or more "peptide barcodes" unique to that particular Fv or to a small subset of Fv's from within the complex library. Preferably, the peptide barcode is C terminal to the single-chain Fv' region or C terminal to the VH or VL or both and includes, flanked between itself and the Fv region, one or more protease sensitive sites such as sites for enterokinase (cleaves after Asp-Asp-Asp-Asp-Lys, Factor Xa (cleaves after Ile-Glu/Asp-Gly-Arg) or other endopeptidases. If a mixture of such Fv's is produced from a suitable gene library, then this mixture is mixed with a target antigen (or antigens such as on cells), usually where the antigen is immobilised. This results in specific Fv's binding to the target antigen with non-binders (or weak binders depending on the stringency of washing) being washed away. Having washed away excess antibodies, the remaining antigen/Fv complex is then usually released from the Fv by digestion with the endoprotease used to cleave the introduced protease sensitive site. This released barcoded peptide is then subjected to mass analysis / mass spectrometry sequencing either directly or, if desired, following capture by virtue of specific amino acids or amino acid sequences which allow the peptide to be captured onto a solid phase such a cysteine residues which can be biotinylated for subsequent capture on immobilised avidin or streptavidin. Alternatively, any other method can be employed to determine the sequence of the peptide barcode either within the Fv or after release including using specific ligands which bind to the barcode in a sequence-specific manner. Having determined the sequences (or part-sequence) of barcodes derived from bound Fv's, corresponding synthetic oligonucleotides are then produced and used to specifically amplify or enrich for specific Fv genes from the library. These specific or enriched Fv (or VH and VL) genes are then further used to generate corresponding Fv's which could then be retested for antigen binding either individually or as part of a small pool of isolated Fv's. Ultimately, by this method, specific Fv's can be generated with desirable antigen binding properties and, if from a human source, potential clinical utility. This aspect also encompasses the use of multiple barcodes associated with individual proteins or Fv's, for example two adjacent barcodes at the C terminus of Fv's whereby two peptides are released from each Fv by protease digestion, either simultaneously in order to enhance the identity of Fv's which bind to the target, or sequentially whereby different proteases are used in successive rounds of digestion to provide a different means to subsequently amplify Fv genes corresponding to Fv's which bind to the target. This aspect also encompasses the use of multiple barcodes which are analysed at the same time in order to increase the diversity of overall barcode sequences to provide specific coding of individual proteins. This aspect also encompasses the use of barcodes within individual proteins, for example within one or more CDR positions of an Fv. This aspect also encompasses the use of proteases which might also digest the protein components of the protein:target mixture or, additionally, any protein agent used to immobilise the target, with the proviso that the barcode peptides released from the bound test protein(s) can still be detected and sequenced within the background of other peptides. In the preferred format of this aspect, a single region of barcode is provided at the C terminus of the light chains forming a soluble Fab fragment whereby VHs and VLs are encoded by the same expression cassette or cistron such that the barcode sequence can be used to access both VH and VL genes. Such an Fab fragment can be conveniently produced using a range of expression systems, for example the M13 bacteriophage vector system where, by introduction of secretory leader sequences, the heavy and light chains of Fabs are secreted into the periplasmic space of the host bacteria and harvested from that space. The vector system is first prepared with in-frame barcodes by cloning in mixtures of synthetic oligonucleotides. For the formation of two adjacent barcodes, this is conveniently undertaken by sequential cloning or oligonucelotide mutagenesis whereby pooled M13 recombinants containing the first mixed barcode are prepared as a template for subsequent cloning of the second in-frame barcode. Preferably, the barcoding is designed such that the encoded protein contains endonuclease sites both flanking and between the two barcodes and also whereby a "spacer" region adjacent to one of the barcodes creates a peptide including that barcode which has a higher molecular weight than the other barcode. By judicious design of barcodes and the use of multiple barcodes in this manner, there is provided an option to simply analyse masses of endoprotease-released peptides by, for example, MALDI-ToF whereby the sequences of the peptides can be deduced (or near deduced) such that synthetic oligonucleotides can be designed to isolate (or enrich) for the specific proteins with the barcode(s) detected by MALDI-ToF analysis. Such deduction of these sequences is achieved by design of sequences whereby specific amino acids only occur in one or two positions along the peptide. For example, where the peptide is designed using 17 of the 20 natural amino acids (hereby designated A-Q), then the sequences might be designed with options for any of three amino acids at each position along the peptide sequence as follows;

| | | | | | | | | |
|---|---|---|---|---|---|---|---|---|
| aa position: | 1 | 2 | 3 | 4 | 5 | 6 | 7 | 8 |
| amino acid options: | A | C | E | G | I | K | M | O |
| | B | D | F | H | J | L | N | P |
| | C | E | G | I | K | M | O | Q |

This design would give a theoretical 6561 different peptide sequence barcodes. If an adjacent barcode with a spacer region is also designed on the same basis, then this would give an additional 6561 different barcodes. In combination, this would create 4 x 10⁷ barcode sequences which would be adequate to uniquely tag most members of a protein library of such size. The use of additional adjacent barcodes or longer barcodes based, for example, on use of two specific amino acids at any position in the sequence (thus creating 262,144 different barcode sequences using 19 amino acids) would increase the diversity of barcodes provided. In practice, codon redundancy is reduced through the judicious choice of codons at each position in the sequence during design of mixed synthetic oligonucleotides. One design of oligonucleotide for an 8 amino acid barcode peptide for MS/MS sequencing is as follows;

| | | | | | | | | |
|---|---|---|---|---|---|---|---|---|
| Codons - | NAC | NCC | NGG | NTG | TKC | VAG | GNV | CNT |
| Amino acids - | N | T | R | L | F | Q | D | H |
| | D | P | G | M | C | E | V | L |
| | H | A | W | V | | K | A | P |
| | Y | S | | | | | G | R |

| | | | | | | | | |
|---|---|---|---|---|---|---|---|---|
| where codons N = A, C, G or T | | | | | | | | |
| K = G or T | | | | | | | | |
| V = A, C or G | | | | | | | | |
| 4 X 4 X 3 X 3 X 2 X 3 X 4 X 4 = 13824 barcode sequences | | | | | | | | |

Specific codons can also be incorporated by discontinuous oligonucleotide synthesis whereby specific codons are added sequentially to separated mixtures of previously synthesised oligonucleotides ("codon mutagenesis''). Once a candidate barcode sequence is deduced by MALDI-ToF or MS/MS and where the diversity of individual barcodes is less than that of the library, the corresponding specific oligonucleotide (or mixture of oligonucleotides if there is redundancy in codon usage) can be used as a PCR primer in conjunction with an opposite primer designed from the protein or vector system to enrich for genes encoding the protein from which the barcode was detected. Where adjacent barcodes are used, a second primer nested within a gene fragment created from the first primer can then be used to enrich for the gene encoding the actual protein detected. If required, the above method can incorporate three or more barcodes in order to increase the specificity of oligonucleotide-directed enrichment of specific genes encoding the desired protein. It will be understood by those skilled in the art that this first aspect of the invention can cover a number of variant methods with the underlying principle that a specific protein is recovered from a library of such proteins via mass or sequence analysis of one or more peptides associated with or encoded by that specific protein and, as such, that this aspect has a broad utility in isolating genes encoding desired proteins where only peptide sequence is determined or deduced.

It will be understood by those skilled in the art that, within the scope of the present invention, there are many variations of the first aspect. For example, it will be understood that peptide barcodes could be incorporated into pairs or groups of proteins which are then allowed to bind in order to determine which proteins binds to each other by virtue of detecting barcodes from each of the proteins engaged in binding. As an alternative to isolation of proteins from complex mixtures, proteins within these complex mixtures which demonstrate certain binding properties such as binding to other macromoles such as DNA can be detected using the present method. The present method includes a variety of ways for adding peptide barcodes to proteins including methods where the barcode is encoded within the gene fragment encoding the protein. However, barcodes can be added to such proteins or to any other suitable mixture of molecules by direct attachment of peptides. For example, specific peptides can be added to specific antibodies or proteins using a range of chemical or photochemical methods. One application of such a method is to labet one complex mixture of proteins with one barcode (or selection of barcodes, for example with different protein specificities) and the other barcode to an alternative complex mixture of proteins, for example to differentially barcode proteins from two different samples which are then mixed. It will be understood by those skilled in the art that the principle of adding peptide barcodes to proteins or other molecules could also be applied to non-peptide barcodes whereby such barcodes can be directly identified (or nearly identified) using mass or sequencing methods. As such, the barcodes could include nucleic acid barcodes attached to proteins or other molecules including nucleic acids. As with peptide barcodes, such nucleic acid barcodes can be analysed by mass spectrometry to provide an accurate estimate of mass. Such barcodes might be released from the proteins or other molecules using restriction enzymes instead of proteases.

It will be understood by those skilled in the art that, within the scope of the present invention, there are applications of the first aspect other than in the isolation of proteins, For example, the distribution of proteins or other ligands within a live organism can be analysed by analysis of barcodes by mass or by sequence which are associated with specific organs within the organism. In the analysis of peptide or protein binding specificity to other molecules, barcodes can be constructed as part of the peptide or protein binding regions in order to analyse specificity by mass or sequence analysis of barcodes. For example, mixed peptide barcode sequences can be constructed around known anchor residues of MHC moelcules and the spectrum of peptides which bind to specific MHC molecules then determined by elution and mass or sequence analysis of the barcode.

The invention is illustrated by the following examples which some not be considering as limiting in scope.

### Example 1

The experiments described in the present example were conducted using a pair of modified single chain antibody (scAbs) genes. Two modified scAbs were prepared consisting of N-terminal epitope tags, the heavy chain variable region (VH), a 14 amino acid linker (EGKSSGSGSESKVD), the light chain variable region (VL) each fused to the b-zip domain from either the c-jun or c-fos genes.

These constructs were cloned into the vector pET 5c (Rosenberg AH et al., Gene, 56:125-135, 1987) which provides a T7 promoter followed by the ribosome binding site from T7 gene 10. The scAb constructs were inserted into the vector at an NdeI site such that the sequence encoding the epitope tag followed the first ATG of T7 gene10. The first construct consisted of a scAb against Pseudomonas aeruginosa (Molloy P. et al. *Journal of Applied Bacteriology,* **78**: 359-365, 1995) with the FLAG epitope (MDYKDDDK) (Knappik A and Pluckthun A, *BioTechniques,***17***:* 754-761,1994) added at the N terminus, and the b-zip domain of c-fos (Abate, C. et al *Proc. Natl. Acad. Sci. USA.* **87:** 1032-1036,1990) at the C-terminal region of the protein. The second consisted a scAb constructed from the anti-foetal antigen antibody 340 (Durrant LG et al. *Prenatal Diagnosis,* **14**:131-140, 1994) with a poly-Histidine tag at the N terminus, and the b-zip domain of c-jun (Abate C. et al, *ibid)* at the C-terminal region of the protein.

The *anti-Pseudomonas aeruginosa* (α-Ps-fos) scAb and the 340-jun scAb were constructed as described below:

DNA for the α-Ps scAb in the vector pPM1His (Molloy P et al., ibid) was amplified with the primers RD 5' FLAG: 5'gcggatcccatatggactacaaagacgatgacgacaaacaggtgcagctgcag3' (Genosys Biotechnologies Europe Ltd, Cambridge, UK) and RD 3': 5'gcgaattcgtggtggtggtggtggtgtgactctcc3' (Genosys) which introduced the 5' FLAG epitope sequence and removed the 3' stop codon respectively. The reaction mixture included 0.1 µg template DNA, 2.6 units of Expand^{™} High Fidelity PCR enzyme mix (Boehringer Mannheim, Lewes, UK.), Expand HF buffer (Boehringer Mannheim), 1.5 mM MgCl₂, 200 µM deoxynucleotide triphosphates (dNTPs) (Life Technologies, Paisley, UK) and 25 pmoles of each primer. Cycles were 96°C 5 minutes, followed by [95°C 1 minute, 50°C 1 minute, 72°C 1 minute] times 5, [95°C 45 seconds, 50°C 1 minute, 72°C 1 minute 30 seconds] times 8, [95°C 45 seconds, 50°C 1 minute, 72°C 2 minutes] times 5, finishing with 72°C 5 minutes. The 1123 bp product obtained was cut with BamHI and EcoRI and cloned into the vector pUC19 (Boehringer Mannheim). The DNA sequence was confirmed, using the Thermo Sequenase radiolabeled terminator cycle sequencing kit with [³³P] dideoxy nucleotides (Amersham Life Science, Amersham, UK). The construct was cloned into pET5c vector (Promega UK Ltd, Southampton, UK.) as a NdeI to EcoRI fragment (see *Molecular Cloning, A Laboratory Manual* eds. Sambrook J, fritsch EF, Maniatis T. Cold Spring Harbor Laboratory Press 1989, New York, USA). Plasmid DNA was prepared using Wizard® Plus SV Minipreps DNA purification System (Promega UK Ltd), or for larger scale, Qiagen Plasmid Midi Kit (Qiagen Ltd, Crawley, UK.). The new plasmid generated was named pET5c FLAG-αPs scAb.

The fos cassette was assembled by PCR of overlapping oligonucleotides:
Fos1for 5'-atggaattcctcgagaccgacaccctacaggcggaaaccgaccagctgga
Fos80rev 5'-tcgcgatttcggtttgcagcgcggatttttcgtcttccagctggtcggtt
Fos 71for 5'-aaaccgaaatcgcgaacctgctgaaagaaaaagaaaagctggagttcatc
Fos 155rev 5'ggaagcttgaattccgccggacggtgtgccgccaggatgaactccagctt

The above oligonucleotides were included in a reaction mix at 1pmol each, and the reaction was driven using 10pmol primers Fos1fS; 5'-atggaattcctcgagacc and Fos 155rS 5'-ggaagcttgaattccgcc using high fidelity polymerase and reaction components as previously. The resulting 155bp product was digested with EcoRI, purified and cloned into EcoRI cut pUC19 for sequence analysis using standard procedures (see *Molecular Cloning, A Laboratory Manual* ibid). The Fos cassette was sub-cloned into the pET5c FLAG-αPs scAb plasmid as an XhoI-EcoRI fragment by substitution of the existing 320bp XhoI-EcoRI fragment carrying the human constant region domain.

The 340 scAb was produced by substitution the VH and VK of the 340 antibody in place of the α-Ps VH and VK in ppMIHis. The 340 VH was amplified with the primers 5'cagctgcaggagtctgggggaggcttag3' (Genosys) and 5'tcagtagacggtgaccgaggttccttgaccccagta3' (Genosys). The reaction mixture included 0.1µg template DNA, 2.6 units of Expand^{™} High Fidelity PCR enzyme mix, Expand HF buffer, 1.5 mM MgC12,200 µM dNTPs and 25 pmoles of each primer. Cycles were 96°C 5 minutes, followed by [95°C 1 minute, 50°C 1 minute, 72°C 1 minute] times 5, [95°C 45 seconds, 50°C 1 minute, 72°C 1 minute 30 seconds] times 8, [95°C 45 seconds, 50°C 1 minute, 72°C 2 minutes] times 5, finishing with 72°C 5 minutes. The 357 bp product was cut with PstI and BstEII and cloned into PstI and BstEII cut pPM1His (see *Molecular Cloning, A Laboratory Manual,* ibid). Similarly, the 340 VK was amplified with the primers 5'gtgacattgagctcacacagtctcct3' and 5'cagcccgttttatctcgagcttggtccg3' (Genosys). The 339 bp product was cut with SstI and Xhol and cloned into SstI and XhoI cut modified pPM1His (produced above). The DNA sequence was confirmed, using the Thermo Sequenase radiolabeled terminator cycle sequencing kit with [³³P] dideoxy nucleotides as before. DNA for the 340 scAb in the vector pPM1His was amplified with the primers RD 5' HIS: 5'gcggatcccatatgcaccatcatcaccatcaccaggtgcagctgcag3 (Genosys) and RD 3' (given above) which introduced the 6 histidine residues at the 5' end and removed the 3' stop codon respectively. Reagents and conditions for amplification were exactly as for the α-Ps construct. The 1114 bp product obtained was cut with BamHI and EcoRI and cloned into the vector pUC19 (see *Molecular Cloning. A Laboratory Manual,* ibid). The DNA sequence was confirmed as before and the construct was cloned into pET5c vector as a NdeI to EcoRI fragment to generate the plasmid pEt5c HIS 340 scAb.

The jun cassette was assembled by PCR of overlapping oligonucleotides:
Junlfor 5'-atgagaattctcgagcgtatcgctcgtctggaagaaaaagttaaaaccct
Jun 85rev 5'-tagcggtggaagccagttcggagttctgagctttcagggttttaactttt
Jun 71for 5'-tggcttccaccgctaacatgctgcgtgaacaggttgctcagctgaaacag
Jun 146rev 5'-catgcgaattcgtggttcataactttctgtttcagctgagcaacc

The above oligonucleotides were included in a reaction mix at 1pmol each, and the reaction was driven using 10pmol primers Jim 1 for-S; 5'-atgagaattctcgagcg and Jun 146rev-S; 5'-catgcgaattcgtggttc using high fidelity polymerase and reaction components as previously. The resulting 146bp product was digested with EcoRI, purified and cloned into EcoRI cut pUC19 for sequence analysis using standard procedures (see *Molecular Cloning, A Laboratory Manual* ibid). The Jun cassette was sub-cloned into the pEt5c HIS 340 scAb plasmid as an XhoI-EcoRI fragment by substitution of the existing 320bp XhoI-EcoRI fragment carrying the human constant region domain

Plasmids his-340-jun and FLAG-αPs-fos, were used as templates for PCR using biotinylated primer BioT7; 5'-agatctcgatcccgcaaatta and primer petrev;-5'-aaataggcgtatcacpggcc. Primers were supplied by GenoSys (Cambridge, UK) and used in the reaction at a concentration of 1pmoL Components and PCR conditions were as previously. The his-340-jun reaction product was 992bp, and the FLAG-αPs-fos reaction product was 1002bp. The products were purified using a spin purification cartridge (Qiagen, Crawley, UK) and diluted to 100ng/µl concentration. Quantitation was by UV absorbance at 260nm. 500ng biotin labelled DNA was reacted with 10µl streptavidin coated magnetic particles (Bangs labs, Fishers, USA). The reaction was conducted in a siliconised microcentrifuge tube in a volume of 500µl PBS 1% (w/v) BSA for 10 minutes at room temperature. Following binding, the particles were collected by magnet (Dynal, Bromborough, UK) and washed three times using PBS 1% BSA.

Following the final wash, *in vitro* translation reaction was initiated by addition of 25µl T7 Quick coupled transcription translation mix (Promega, Southampton, UK) supplemented with biotinyl lysine tRNA (Promega). The translation reaction was conducted at 30°C for 60 minutes then placed on ice. Particles were collected by magnet, and washed using ice cold PBS containing 1% BSA.

In some experiments, non-magnetic steptavidin particles were used in IVTT reactions (Bangs Labs, Fishers, USA). In such cases particles were recovered during wash cycles by centrifugation.

In some experiments, coloured streptavidin particles, magnetic and non magnetic (Bangs Labs), were used in IVTT reactions.

In some experiments translation products bound to the particles were detected using antibodies for either the Flag or the his6 epitope engineered into each of the model gene constructs. Antibodies were added to the washed particles diluted in PBS. Incubations were for 60 minutes at 4°C with gentle mixing. A secondary reagent (anti-mouse-HRP conjugate) was added at the recommended dilution in PBS and incubated for a further 30 minutes at 4°C. Particles were washed three times using 200µl PBS before colour development with the chromogenic substrate. Reactions were read at 492nm.

Protein-protein binding reactions were conducted using IVTT proteins bound to the particle surface. In such experiments, non magnetic streptavidin particles were "captured" by protein mediated (fos:jun) binding to the surface of magnetic particles. Magnetic particles with fos IVTT product were mixed gently with non magnetic particles with jun bound on the surface. The reaction was conducted in 100µl PBS, BSA and allowed to proceed at room temperature for 30 minutes. In a negative control reaction, non-magnetic particles with a Sea protein (a-Ps scAb; Molloy P et al., ibid) bound on the surface were mixed with the magnetic particles coated with the fos IVTT product. Following incubation, the particles were captured by magnet and washed six times using PBS, 1% BSA.

The presence of the captured target protein gene was confirmed using PCR and DNA sequencing. For detecting the jun model gene, jun specific primers Jun 1for-S and Junl46rev- were used in a PCR assay. The assay was initiated by addition of 10% (v/v) particles directly into the PCR mix. Components and reaction conditions were as previously. The 146bp jun specific product was detected by gel electrophoresis. For detecting the fos model gene, primers FoslfS and Fos 155rS were used in a PCR assay. Reaction conditions and detection of the 155bp fos specific product were as above. For detecting the negative control protein, primers Seqlscab 5'agatccctactataggta and Seq2scab; 5'-ggtgagctcgatgtatcc were used to detect a 115bp product in the α-Ps scAb protein gene.

In the above experiments, Jun PCR products were detected following capture by fos magnetic particle under conditions were no α-Ps scAb PCR products could be detected following interaction with the fos magnetic particles.

### Example 2

In this example a single-chain antibody library was produced including unique peptide "barcodes". Human peripheral blood lymphocyte RNA was prepared according to standard procedures. Briefly, lymphocytes were prepared from 10ml heparinised blood taken from 16 normal healthy donors. Lymphocytes were collected following a density gradient centrifugation procedure using Lymphoprep medium (Sigma, Poole, UK). RNA was prepared using the QuickPrep system and instructions provided by the supplier (Pharmacia, St Albans, UK). Synthesis of cDNA was conducted using a cDNA synthesis kit (Pharmacia, St Albans, UK) and random hexamer primers with conditions recommended by the supplier. Immunoglobulin heavy chain variable region (Vh) and light chain variable regions (Vl) were amplified from cDNA in separate PCR mixes using primer sets designed to maximise Vh and Vl repertoires. Primer sets were as described previously (Marks J.D. et al 1991, Eur. J. Immunol. 21: 985). Vh and Vl PCR reactions were conducted using, 2.6 units of Expand™ High Fidelity PCR enzyme mix (Boehringer Mannheim, Lewes, UK.), Expand HF buffer (Boehringer), 1.5 mM MgCl₂, 200 µM deoxynucleotide triphosphates (dNTPs) (Life Technologies, Paisley, UK) and 25 pmoles of each primer pool. Cycles were 96°C 5 minutes, followed by [95°C 1 minute, 50°C 1 minute, 72°C 1 minute] times 5, [95°C 45 seconds, 50°C 1 minute, 72°C 1 minute 30 seconds] times 8, [95°C 45 seconds, 50° C 1 minute, 72°C 2 minutes] times 5, finishing with 72°C 5 minutes.

In a separate PCR, a linker fragment of form (Gly₄Ser)₃ (Huston J.S. et al 1988, *PNAS,* 85: 5879-5883) was amplified from a cloned template pSW1-ScFvD1.3 (McCafferty et al, 1990, *Nature* **348**: 522-554) using primers sets detailed previously (Marks, J. D in *Antibody Engineering,* ed Borrebaek C.A.K New York O.U.P.,1995). The 93bp linker fragment product was annealed together with an equimolar mixture of the Vh and Vl PCR products. The mixture was further amplified in a "pull through" reaction using flanking primers HuVHBACKsfi and HuFORNot as detailed in Vaughan et al (Vaughan T.J. et al 1996, *Nature Biotech.* **14**: 309-314). All fragments used in the pull-tlirough reaction were purified free of their initial primers prior to inclusion in the reaction. Purification was conducted using the Wizard PCR Preps system from Promega (Promega, Southampton UK).

The assembled contig of form Vh-linker-Vl, was digested with restriction enzymes SfiI and NotI (Boehringer) using standard conditions and purified as above. The purified fragment was annealed with a double stranded synthetic oligonucleotide adapter mix designed to introduce a V8 protease cleavage site juxtaposed with a tract of randomised sequence in frame with the C-terminus of the V1 gene. This V8/unique sequence barcode was produced by annealing a pair of synthetic oligonucleotide pools of form 5'-ggccgcgaggaagaggaa[(atg)/(can)/(agn)/(aan)/(gan)/(ttn)]₂gc-3' and 5'-ggccgc[(naa)/(ntc)/(ngt)/(nct)(nag)/(cat)]₂ctccttctcctcgc-3'. This linker has NotI compatible ends (underlined) and therefore facilitates the insertion of the complete single chain antibody-V8/unique sequence barcode fragment into SfiI-NotI prepared pCANTAB 5 (Pharmacia) phagemid vector.

The unique sequence barcode was designed to avoid the introduction of stop codons and further biased to exclude encoding residues with greater than two alternative codons. By this strategy, the number of specific oligonucleotides required to identify a given de-coded peptide sequence, is minimised. In all, the unique sequence barcode is able to encode 11 of the 20 amino-acids. In addition to the V8 peptidase cleavage site (a string of 4 glutamic acid residues), the sequence barcode is 12 codons long. Thus from the repertoire of 11 amino acids (10 of which are encoded by either of two codons), is able to encode 11 ¹²/2 = ~1.5x10¹² different peptides.

The assembled scfv fragment (Vh-linker-VI) with SfiI and NotI prepared ends was annealed and ligated to the NotI sequence-barcode adapter and re-purified. For experiments expressing the human scfv library by phage display, the complete fragment was ligated into SfiI-NotI prepared pCANTAB 5 (Pharmacia) phagemid vector, and transformed into competent TG1 E.coli.

For other experiments using *in vitro* transcription and translation (IVTT), the assembled scfv library was subcloned into SfiI NotI prepared pCANTAB5-T7. This vector is the same as the commercially available pCANTAB5 except it was modified to include the T7 promoter sequence (ttaatacgactcactata) inserted at the HindIII site at position 2235. The modification was achieved by ligation of a double-stranded synthetic DNA linker of sequence 5'- agctaatacgactcactata into HindIII cut and dephosphorylated pCANTAB5. Recombinant clones containing the T7 promoter were selected using a diagnostic PCR.

Following ligation and transformation into competent TG1 E.coli, cells were grown for 1 hour in 1ml of SOC medium and then plated onto TYE medium with 100ug/ml ampicillin. Colonies were scraped off plates into 5ml of 2x TY broth containing ampicillin. The cultured library was used to prepare DNA for IVTT reactions.

The pCANTAB5-T7 Sefv library DNA was used in an *in vitro* translation reaction. The IVTT was conducted using the T7 Quick coupled transcription translation mix (Promega, Southampton, UK) and 10µg of the pCANTAB5-T7 Scfv library DNA in a total volume of 50µl The translation reaction was conducted at 30°C for 90 minutes then placed on ice. In some experiments reactions were monitored for the presence of translation products using ³⁵S-methionine incorporation assays. Reactions were stored at -70°C prior to use in binding and screening assays.

The single-chain antibody library was used to in a binding reaction to recombinant human p53 protein (Oncogene Resarch Products-Calbiochem,Nottingham, UK). The IVTT mix was diluted x10 fold in PBS and used in a binding assay to human recombinant p53 protein immobilised in a 96-well microplate. The p53 protein was immobilised by overnight incubation at a concentration of 100µg/ml in phosphate buffer at 4°C. The plate was washed using PBS 0.5% (w/v) BSA and the diluted IVTT mix added to the test and control wells for binding. The binding reaction was conducted at 37°C for 90 minutes. The plate washed x3 using PBS-T (PBS + 0.05% v/v tween-20) and subjected to V8 protease digestion (Takara, Wokingham, UK). Protein fragments were collected from the supernatant and size fractionated to exclude the V8 protease and other large species before analysis by MALDI-tof.

MALDI-tof fragment analysis identified a number of peptide fragments. The peptide sequences were used to design a set of corresponding synthetic oligonucleotides. The oligonucleotides were used in a PCR based screen of the single chain library. Pfn turbo (Stratagene Europe) DNA polymerase was used to synthesise complementary strands in members of the human single-chain antibody library DNA. Following 15 rounds of thermal cycling, the product was subjected to DpnI digestion. This step depleted the mixture of parental plasmid molecules to ensure that only the newly synthesised primed products were propagated. 1µl of the reaction was transformed into TG1 competent cells and plated onto LB plates containing 100µg/ml ampicillin. Individual clones were picked, expanded and DNA prepared according to standard procedures. The DNA was used directly in a second round of screening involving IVTT, antigen binding, V8 protease digestion, MALDI-tof fragment analysis. After 2 rounds of selection 6 scFv's were isolated which bound recombinant p53.

## Claims

1. A library of individual proteins, one or more of which being able to bind to a target of interest, each of said proteins comprising within its amino acid sequence or terminal to it one or more synthetic individual identifier sequence tract(s) which are "barcode" sequences, which are unique to the particular protein bound to the specific target of interest, and are flanked by one or more protease sensitive sites, said library thus providing proteins comprising a diversity of said synthetic individual identifier sequence tracts.

2. A library of claim 1, wherein said identifier sequence tracts have been generated randomly or semi-randomly.

3. A library of claim 1 or 2, wherein an individual protein of said library comprises multiple identifier sequence tracts.

4. A library according claim 3, wherein an individual protein comprises two adjacent identifier sequence tracts.

5. A library according to any of the claims 1-4, wherein said protease sensitive site is a recognition site for endoprotease digestion.

6. A library of claim 5, wherein the recognition site is the site for enterokinase or Factor Xa.

7. A library according to any of the claims 1-6, wherein said target of interest is a protein.

8. A library of any of the claims 1-7, wherein the individual proteins of the library are antibodies or recombinant antibody domains, which comprise antibody variable regions.

9. A library of claim 8, wherein the antibody domain is an Fv domain consisting of a VH and a VL chain.

10. A library according to claim 9, wherein each chain has its own identifier sequence tract.

11. A library according to any of the claims 8 - 10, wherein said identifier sequence tract is C-terminal to the Fv domain sequence.

12. A library according to any of the claims 8 -11, wherein the target of interest is an antigen.

13. A method of identification of or screening for a protein which binds to a target of interest wherein the protein is a member of the library as defined in any of the claims 1 -12, comprising the following steps:
(i) bringing each of the individual proteins of the library comprising said one or more synthetic individual identifier sequence tracts and said protease sensitive site(s) in contact or association with one or more of said targets of interest,
(ii) isolating the complex formed by the individual protein and the target of interest,
(iii) digesting said complex to cleave the introduced protease sensitive sites releasing said synthetic individual identifier sequence tract(s), and
(iv) determining said released sequence tract(s) by which said individual protein is finally identifiable.

14. A method of claim 13, wherein the individual protein has been encoded by a DNA construct comprising nucleic sequences coding for said one or more individual identifier sequence tracts and said protease sensitive site(s).

15. A method of claim 14, wherein said DNA construct is part of a vector system.

16. A method according to any of the claims 13-15, wherein the individual protein and target of interest bind in solution.

17. A method according to any of the claims 13 - 16, wherein the complex of protein / target is isolated by removal of non-bound molecules.

18. A method according to any of the claims 13 - 17, wherein digestion of said complex is achieved by endoprotease.

19. A method according to any of the claims 13-18, wherein determination of the released identifier sequence tract(s) is achieved by mass spectrometry.

20. A method of claim 19, wherein the mass spectrometry is MALDI-ToF.

21. A method according to any of the claims 13 - 20, wherein the individual proteins of the library are antibodies or antibody domains having a variable region and the targets of interest are antigens.

22. A method for amplification and expression of the genes encoding the individual protein as identified by a method of any of the claims 13 - 21 by using the nucleic sequence of the synthetic identifier sequence tract as a PCR primer in conjunction with the opposite primer designed from said individual protein or vector system.

## Patentansprüche

1. Bibliothek von einzelnen Proteinen, von denen eines oder mehrere in der Lage sind, an ein Ziel von Interesse zu binden, wobei jedes der Proteine innerhalb seiner Aminosäuresequenz oder an deren Ende eine oder mehrere synthetische individuelle Identifizierungssequenzabfolge(n) umfasst, wobei diese "Barcode"-Sequenzen für das bestimmte, an das spezifische Ziel von Interesse gebundene Protein einzigartig sind und von einer oder mehreren Protease-sensitiven Stellen flankiert sind, so dass die Bibliothek somit Proteine bereitstellt, die eine Vielfalt dieser synthetischen individuellen Identifizierungssequenzabfolgen umfassen.

2. Bibliothek nach Anspruch 1, wobei die Identifizierungssequenzabfolgen zufallsgemäß oder halb-zufallsgemäß erzeugt wurden.

3. Bibliothek nach Anspruch 1 oder 2, wobei ein einzelnes Protein der Bibliothek mehrere Identifizierungssequenzabfolgen umfasst.

4. Bibliothek nach Anspruch 3, wobei ein einzelnes Protein zwei benachbarte Identifizierungssequenzabfolgen umfasst.

5. Bibliothek nach einem der Ansprüche 1 - 4, wobei die Protease-sensitive Stelle eine Erkennungsstelle für die Endoprotease-Spaltung ist.

6. Bibliothek nach Anspruch 5, wobei die Erkennungsstelle die Stelle für Enterokinase oder Faktor Xa ist.

7. Bibliothek nach einem der Ansprüche 1 - 6, wobei das Ziel von Interesse ein Protein ist.

8. Bibliothek nach einem der Ansprüche 1-7, wobei die einzelnen Proteine der Bibliothek Antikörper oder rekombinante Antikörperdomänen sind, die variable Antikörperregionen umfassen.

9. Bibliothek nach Anspruch 8, wobei die Antikörperdomäne eine Fv-Domäne ist, die aus einer VH- und einer VL-Kette besteht.

10. Bibliothek nach Anspruch 9, wobei jede Kette ihre eigene Identifizierungssequenzabfolge besitzt.

11. Bibliothek nach einem der Ansprüche 8 - 10, wobei sich die Identifizierungssequenzabfolge C-terminal der Fv-Domänensequenz befindet.

12. Bibliothek nach einem der Ansprüche 8 - 11, wobei das Ziel von Interesse ein Antigen ist.

13. Verfahren zur Identifizierung oder zum Screening eines Proteins, das an ein Ziel von Interesse bindet, wobei das Protein ein Mitglied der Bibliothek nach einem der Ansprüche 1-12 ist, das die folgenden Schritte umfasst:
(i) Zusammenbringen oder Binden jedes der einzelnen Proteine der Bibliothek, die die einen oder mehreren synthetischen individuellen Identifizierungssequenzabfolgen und die Protease-sensitive(n) Stelle(n) umfassen, mit/an ein(em) oder mehrere(n) der Ziele(n) von Interesse,
(ii Isolieren des Komplexes, der von dem einzelnen Protein und dem Ziel von Interesse gebildet wird,
(iii) Spalten des Komplexes, so dass die eingeführten Protease-sensitiven Stellen gespalten und die synthetische(n) individuelle(n) Identifizierungssequenzabfolge(n) freigesetzt wird/werden, und
(iv) Bestimmen der freigesetzten Identifizierungssequenzabfolge(n), über die das einzelne Protein schließlich identifiziert werden kann.

14. Verfahren nach Anspruch 13, wobei das einzelne Protein von einem DNA-Konstrukt kodiert wird, das Nucleinsäuresequenzen umfasst, die für einen oder mehreren individuellen Identifizierungssequenzabfolgen und die Protease-sensitive(n) Stelle(n) kodieren.

15. Verfahren nach Anspruch 14, wobei das DNA-Konstrukt Teil eines Vektorsystems ist.

16. Verfahren nach einem der Ansprüche 13 - 15, wobei das einzelne Protein und das Ziel von Interesse in Lösung aneinander binden.

17. Verfahren nach einem der Ansprüche 13 - 16, wobei der Komplex aus Protein/Ziel durch Entfernen ungebundener Moleküle isoliert wird.

18. Verfahren nach einem der Ansprüche 13 - 17, wobei die Spaltung des Komplexes durch Endoprotease erreicht wird.

19. Verfahren nach einem der Ansprüche 13 - 18, wobei die Bestimmung der freigesetzten Identifizierungssequenzabfolge(n) mittels Massenspektrometrie erfolgt.

20. Verfahren nach Anspruch 19, wobei es sich bei der Massenspektrometrie um MALDI-ToF handelt.

21. Verfahren nach einem der Ansprüche 13-20, wobei die einzelnen Proteine der Bibliothek Antikörper oder rekombinante Antikörperdomänen mit einer variablen Region und die Ziele von Interesse Antigene sind.

22. Verfahren zur Amplifikation und Expression der Gene, die für das einzelne Protein, wie durch ein Verfahren nach einem der Ansprüche 13-21 identifiziert, kodieren, unter Verwendung der Nucleinsäuresequenz der synthetischen Identifizierungssequenzabfolge als einen PCR-Primer in Verbindung mit dem gegenüberliegenden Primer, der anhand des einzelnen Proteins oder des Vektorsystems gestaltet wird.

## Revendications

1. Bibliothèque de protéines individuelles dont une ou plusieurs peuvent se lier sur une cible digne d'intérêt, chacune desdites protéines comprenant, à l'intérieur de sa séquence d'acides aminés ou en fin de celle-ci, une ou plusieurs portion(s) de séquence d'identificateur individuel synthétique, lesquelles séquences "code barres" sont uniques pour la protéine particulière liée sur la cible digne d'intérêt spécifique et sont flanquées par un ou plusieurs sites sensibles à la protéase, ladite bibliothèque fournissant par conséquent des protéines comprenant une diversité desdites portions de séquence d'identificateur individuel synthétique.

2. Bibliothèque selon la revendication 1, dans laquelle lesdites portions de séquence d'identificateur ont été générées de façon aléatoire ou semi-aléatoire.

3. Bibliothèque selon la revendication 1 ou 2, dans laquelle une protéine individuelle de ladite bibliothèque comprend de multiples portions de séquence d'identificateur.

4. Bibliothèque selon la revendication 3, dans laquelle une protéine individuelle comprend deux portions de séquence d'identificateur adjacentes.

5. Bibliothèque selon l'une quelconque des revendications 1 - 4, dans laquelle ledit site sensible à la protéase est un site de reconnaissance pour une digestion d'endoprotéase.

6. Bibliothèque selon la revendication 5, dans laquelle le site de reconnaissance est le site pour une entérokinase ou Facteur Xa.

7. Bibliothèque selon l'une quelconque des revendications 1 - 6, dans laquelle ladite cible digne d'intérêt est une protéine.

8. Bibliothèque selon l'une quelconque des revendications 1 à 7, dans laquelle les protéines individuelles de la bibliothèque sont des anticorps ou des domaines d'anticorps recombinants, qui comprennent des régions variables d'anticorps.

9. Bibliothèque selon la revendication 8, dans laquelle le domaine d'anticorps est un domaine Fv qui est constitué par une chaîne VH et par une chaîne VL.

10. Bibliothèque selon la revendication 9, dans laquelle chaque chaîne dispose de sa propre portion de séquence d'identificateur.

11. Bibliothèque selon l'une quelconque des revendications 8 - 10, dans laquelle ladite portion de séquence d'identificateur est un terminal-C pour la séquence de domaine Fv.

12. Bibliothèque selon l'une quelconque des revendications 8 - 11, dans laquelle la cible digne d'intérêt est un antigène.

13. Procédé d'identification ou de filtrage d'une protéine qui se lie sur une cible digne d'intérêt, dans lequel la protéine est un membre de la bibliothèque telle que définie selon l'une quelconque des revendications 1 - 12, comprenant les étapes qui suivent :
(i) amenée de chacune des protéines individuelles de la bibliothèque comprenant ladite/lesdites une ou plusieurs portion(s) de séquence d'identificateur individuel synthétique et ledit/lesdits site(s) sensible(s) à la protéase en contact ou en association avec une ou plusieurs desdites cibles dignes d'intérêt ;
(ii) isolation du complexe qui est formé par la protéine individuelle et par la cible digne d'intérêt ;
(iii) digestion dudit complexe afin de cliver les sites sensibles à la protéase introduits en libérant ladite/lesdites portion(s) de séquence d'identificateur individuel synthétique ; et
(iv) détermination de ladite/desdites portion(s) de séquence libérée(s) au moyen de laquelle/desquelles ladite protéine individuelle pour finir peut être identifiée.

14. Procédé selon la revendication 13, dans lequel la protéine individuelle a été codée au moyen d'une construction d'ADN comprenant des séquences nucléiques codant pour ladite/lesdites une ou plusieurs portion(s) de séquence d'identificateur individuel et ledit/lesdits site(s) sensible(s) à la protéase.

15. Procédé selon la revendication 14, dans lequel ladite construction d'ADN est une partie d'un système vectoriel.

16. Procédé selon l'une quelconque des revendications 13 - 15, dans lequel la protéine individuelle et la cible digne d'intérêt se lient en solution.

17. Procédé selon l'une quelconque des revendications 13 - 16, dans lequel le complexe de protéine/cible est isolé au moyen de l'enlèvement de molécules non liées.

18. Procédé selon l'une quelconque des revendications 13 - 17, dans lequel une digestion dudit complexe est réalisée par endoprotéase.

19. Procédé selon l'une quelconque des revendications 13 - 18, dans lequel une détermination de la/des portion(s) de séquence d'identificateur libérée(s) est réalisée au moyen d'une spectrométrie de masse.

20. Procédé selon la revendication 19, dans lequel la spectrométrie de masse est MALDI-ToF.

21. Procédé selon l'une quelconque des revendications 13 - 20, dans lequel les protéines individuelles de la bibliothèque sont des anticorps ou des domaines d'anticorps présentant une région variable et les cibles dignes d'intérêt sont des antigènes.

22. Procédé pour l'amplification et l'expression des gènes codant la protéine individuelle qui est identifiée au moyen d'un procédé selon l'une quelconque des revendications 13 - 21 en utilisant la séquence nucléique de la portion de séquence d'identificateur synthétique en tant qu'amorce PCR en conjonction avec l'amorce opposée conçue à partir de ladite protéine individuelle ou dudit système vectoriel individuel.
